# EUROPEAN PATENT SPECIFICATION

(11) **EP 1 764 114 B1**
(45) Date of publication and mention of the grant of the patent: **18.11.2009**
(21) Application number: 06120449.1
(22) Date of filing: 11.09.2006
(51) Int. Cl.: A61L 2/10, A47K 1/09, A45D 44/18

(54) **Ultraviolet disinfection device**
Vorrichtung zur Ultraviolettdesinfizierung
Dispositif de désinfection par ultraviolets

(30) Priority: 09.09.2005 CN 200511075576
(43) Date of publication of application: 21.03.2007
(73) Proprietor: Yue, Lai Chor, Kowloon Bay, Kowloon, Hong Kong (CN)
(72) Inventor: Yue, Lai Chor, Kowloon Bay, Kowloon, Hong Kong (CN)
(74) Representative: Harrison Goddard Foote

(56) References cited:
- GB-A- 419 929
- US-A- 3 748 094
- US-A- 3 954 407
- US-A1- 2004 025 899
- US-B1- 6 483 119

## Description

The present invention relates to disinfection devices, in particular, but not exclusively, the invention relates to disinfection devices that use ultraviolet light to kill germs.

It is well known that ultraviolet (UV) light of a particular range of wavelength, called germicidal UV light, can destroy germs. The Ultraviolet Disinfection Device of the present invention is a device that kills germs by means of the germicidal UV light that is generated by the ultraviolet lamp. Elongated personal articles, such as toothbrush and chopsticks, can be disinfected by inserting them into the device.

Currently, there are two types of ultraviolet disinfection devices for disinfecting long objects such as toothbrushes and chopsticks. One type uses an enclosed case, with several hooks or other fixtures inside for hanging or holding the objects to be disinfected. The user only need to place the objects properly, close the cover, and turn on the ultraviolet lamp. Disinfection is achieved by illuminating the objects to be disinfected for a certain period of time with the ultraviolet light that is generated by the ultraviolet lamp inside the device. Although this type of device can disinfect effectively, it is not convenient. The user has to spend time opening and closing the cover, as well as hanging or placing the objects properly. The other type of device, which is more popular, uses a simpler design. By inserting the objects to be disinfected into the disinfecting cells of the device through the openings on the top cover of the device, the portions of these objects that are exposed to the ultraviolet light can be disinfected. But that introduces another more serious issue that concerns safety. Since ultraviolet light can leak from the holes through which the objects are inserted for disinfection, it can hurt the eyes of the user if the user looks into the ultraviolet lamp of the device through those holes.

GB419929 discloses apparatus for sterilising drinking glasses by ultraviolet irradiation. The document discloses a housing having a rotatable carousel therein and a door providing access to the carousel.

In view of the above, it is apparent that there exists a need for an ultraviolet disinfection device that provides effective disinfection and is both convenient and safe to use. This invention addresses this need.

It is an aim of embodiments of the present invention to provide an ultraviolet disinfection device for effective disinfection of objects such as toothbrushes and chopsticks. Another aim of embodiments of the present invention is to provide an ultraviolet disinfection device that is both convenient and safe to use. Embodiments of the invention can both reduce leakage of ultraviolet light, thus protecting a user's eyes, and yet maintain good disinfection capability.

A feature of an ultraviolet disinfection device according to embodiments of the invention is a protective shield which surrounds the ultraviolet lamp that generates the germicidal UV light. The said protective shield provides two protective functions. Firstly, it protects the user's eyes by preventing the ultraviolet light generated by the ultraviolet lamp from leaking directly through the openings of the top cover of the ultraviolet disinfection device. Secondly, it protects the ultraviolet lamp, which it surrounds, from being damaged by mechanical forces exerted by any object that is inserted in any of the disinfection cells of the ultraviolet disinfection device.

In a first aspect of the invention there is provided a germicidal device as set out in claim 1 of the appended claims.

The invention has the advantage that the shield protects a users eyes from irradiation by light from the germicidal lamp.

Preferably the shield is configured to provide a sheath around the lamp.

This feature has the advantage that the shield may provide a convient protective function of the lamp, by preventing objects introduced into the device from damaging the lamp.

Preferably the slats are provided with a major face of each slat substantially perpendicular to an axis of the shield.

Preferably the slats are substantially coaxial

An embodiment of the ultraviolet disinfection device of the present invention can be illustrated as comprising the following major components.

a base that is at the bottom of the ultraviolet disinfection device At the bottom of this base is a base bottom cover. Above this base bottom cover is a circuit board Above this circuit board is a base body The top surface of the base body is a platform from where supporting poles protrude upwards There is a pushbutton switch on the base body connected to the circuit board that is inside the base;
a device body that is placed on top of the base body. This device body includes a base ring that is at the bottom of the device body, and a center shell that is on top of the base ring The base ring is preferably translucent to visible light but opaque to UV light,
a reflection cup that is placed inside the device body. This reflection cup includes two connected portions: a reflection cup ring and a reflection cup body;
a top cover, which can be removable, that is above the reflection cup and on top of the center shell This top cover contains disinfection cell holes, which are on top of the disinfection cells. Near the bottom rim of the top cover, there are locking hooks. From the bottom of the top cover near the disinfection cell holes, there are two triggering hooks protruding downwards;
a protective shield that is mounted on top of the supporting poles It features a shield body, preferably cylindrical, with a number of fixing arms protruding horizontally outwards from the top region of the outer surface of the cylindrical body, a stack of horizontal slats, ring shaped if the shield is cylindrical, strategically deployed at non-uniform vertical spacing below the lower rim of the said cylindrical shield body and a number of thin vertical links protruding downwards from the bottom circular rim of the said cylindrical shield body. The said horizontal, thin, ring-shaped circular slats are linked together and kept at strategic vertical spacing by the said thin vertical links;
a germicidal ultraviolet lamp that is fitted inside the shield body by means of a lamp holder,
a protective shield cover is on top of the shield body. It has the same shape as the top portion of the shield body including its fixing arms;
a mechanical triggering device that is placed inside one of the supporting poles This triggering device includes a pivot supporting frame and a pivoted trigger plank

By this simple design, embodiments of the invention can conveniently disinfect personal belongings and promote personal hygiene by simply inserting any long objects such as toothbrushes and chopsticks into a disinfection cell of this device Meanwhile, the protective shield that surrounds the ultraviolet lamp greatly reduces the leakage of ultraviolet light, thus preventing damage to the user's eyes. Yet it still provides enough disinfection power. Thus, in addition to the convenience of usage, this ultraviolet disinfection device also protects the user's health.

Embodiments of the present invention will now be described by way of example with reference to the accompanying drawings in which:
Figure 1 is a top view of a preferred embodiment of this ultraviolet disinfection device;
Figure 2 is an exploded view of the ultraviolet disinfection device that is shown in Figure 1;
Figure 3 is a sectional view along the axis 3-3 of Figure 1;
Figure 4 is an illustration of the mechanism for the ultraviolet disinfection device of Figure 3 to prevent the ultraviolet light generated by the ultraviolet lamp from leaking directly out of the device;
Figure 5 is a sectional elevation view of the protective shield of the ultraviolet disinfection device that is shown in Figure 2;
Figure 6 is a perspective sectional view along the axis 6-6 of Figure 1;
Figure 7 illustrates the operation of the mechanical triggering device that is placed inside one of the supporting poles, which is shown in Figure 6;
Figure 8 illustrates the strength of the ultraviolet light that leaks from a preferred embodiment of the ultraviolet disinfection device right outside the disinfection cell holes with the protective shield removed;
Figure 9 illustrates the strength of the ultraviolet light that leaks from a preferred embodiment of the ultraviolet disinfection device right outside the disinfection cell holes with the protective shield installed;

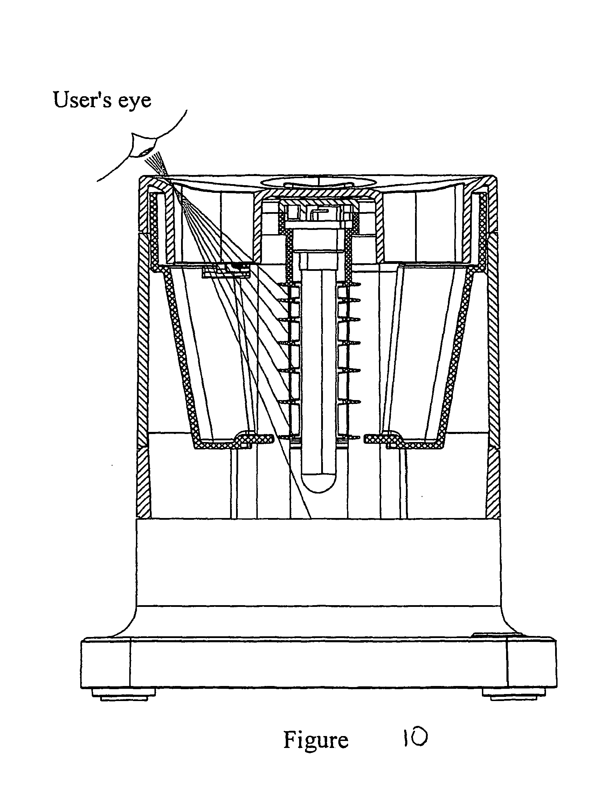

Figure 10 shows a cross section of the ultraviolet disinfection device,

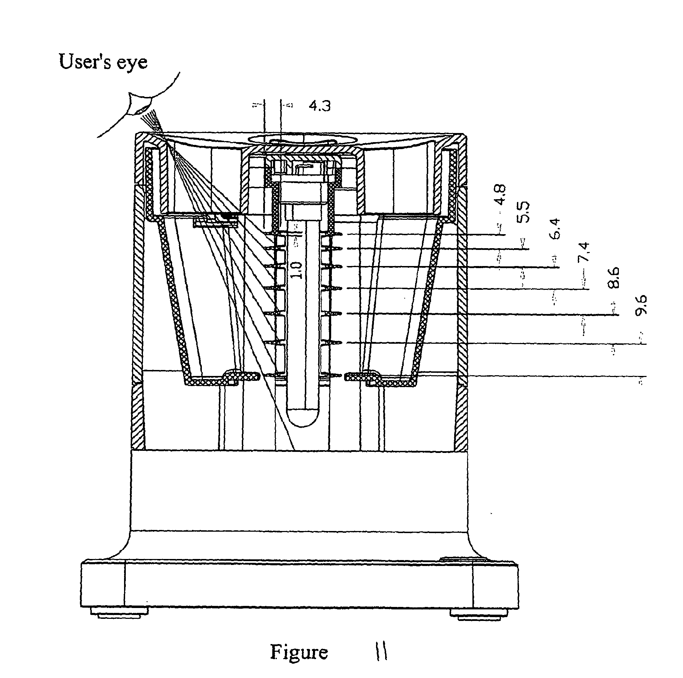

Figure 11 shows a cross section of the ultraviolet disinfection device with an exemplary arrangement of horizontal slats; and

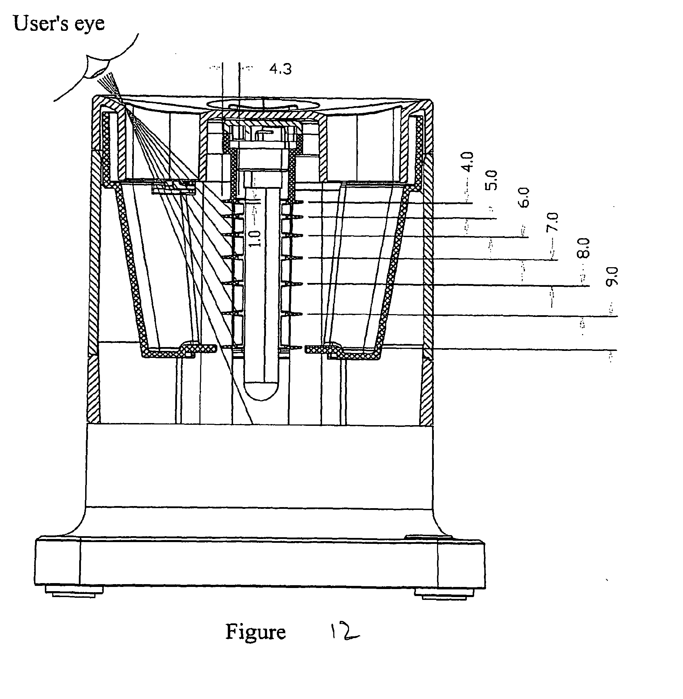

Figure 12 shows a cross section of the ultraviolet disinfection device with another exemplary arrangement of horizontal slats

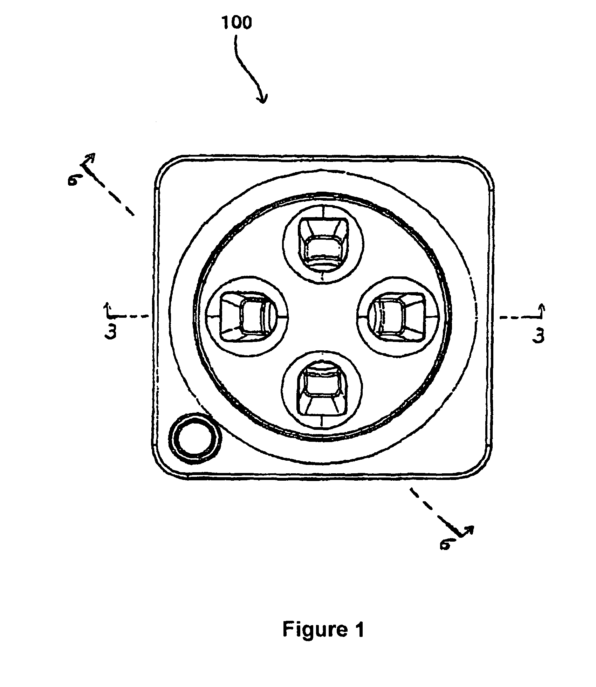

The invention is illustrated through a description of a preferred embodiment in which a device is designed around a cylindrical protective shield. Figure 1 shows the top view of an ultraviolet disinfection device 100 In this embodiment of the invention, there are four disinfection cells 35 and therefore four openings on the top cover of the device

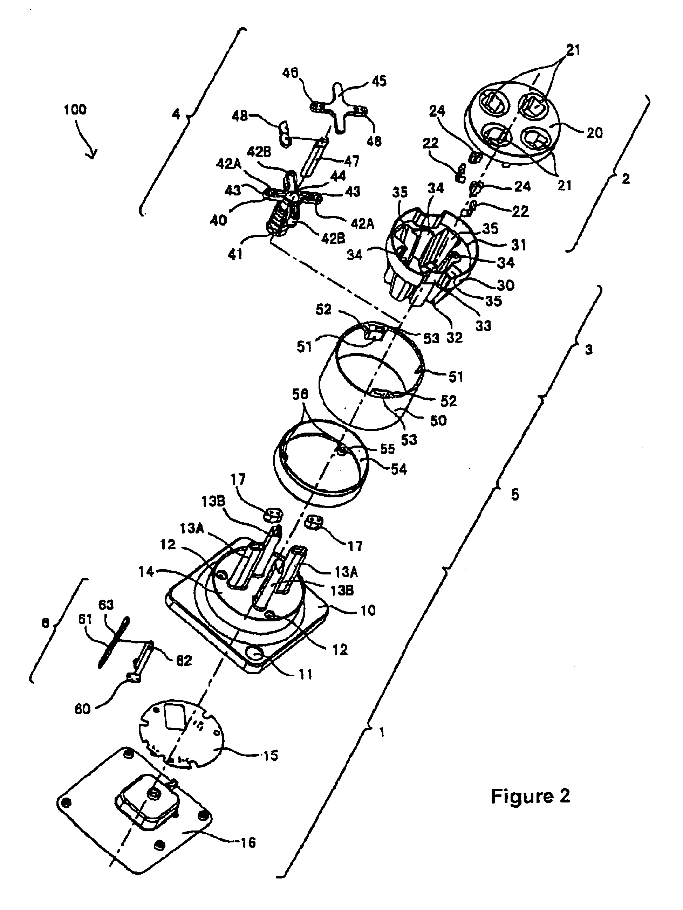

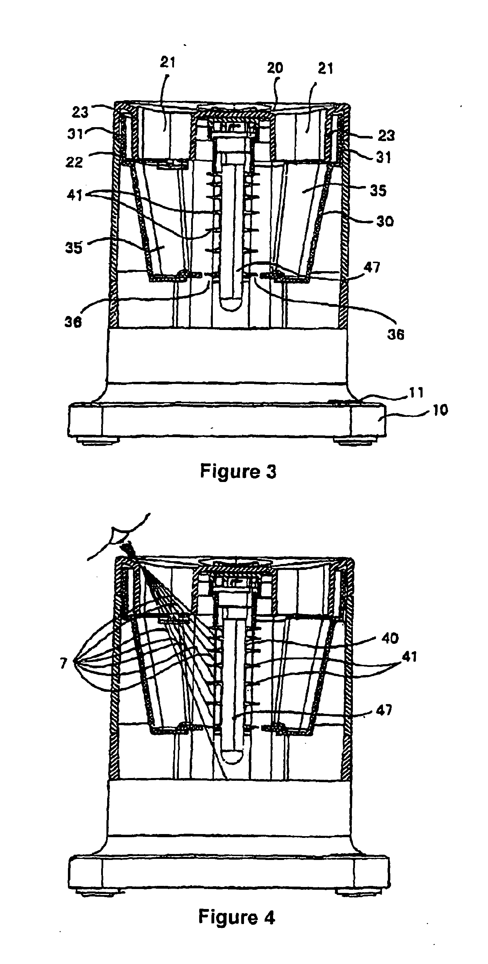

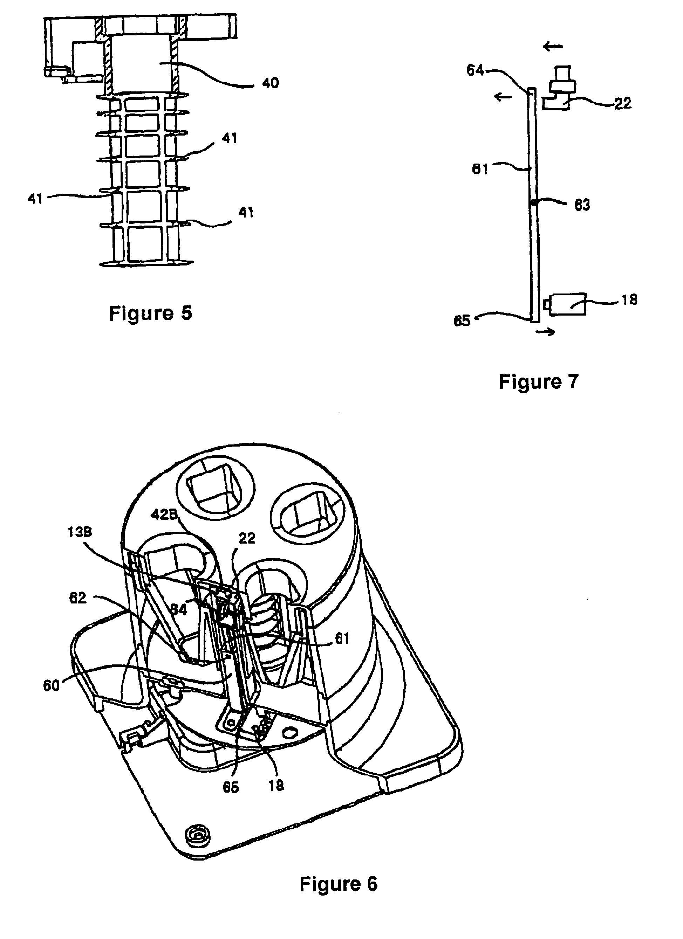

Figure 2 is an exploded view of the preferred embodiment of the ultraviolet disinfection device that is shown in Figure 1. Figure 3 is a sectional view along the axis 3-3 of Figure 1. The device has a base 1 at the bottom of the ultraviolet disinfection device 100 At the bottom of base 1 is a base bottom cover 16 Above the base bottom cover 16 is a circuit board 15. There is a micro-switch 18 inside the base 1 (as shown in Figure 6) The micro-switch 18 is connected to the circuit board 15 Above the circuit board 15 is a base body 10.

The top surface of the base body 10 is raised to form a platform 14 On the platform 14 there are two sets of supporting poles 13A and 13B On top of each supporting pole 13A there is a supporting pole extension 17. Each supporting pole 13B has a triggering hook holding slot on the outer side of the top of supporting pole 13B On the rim of the platform 14 there are several base connection holes 12 to connect to the base ring 54 that is placed inside of platform 14. In one corner of base body 10 there is a pushbutton switch 11 that is connected to the circuit board 15 that is placed inside base 1. A mechanical triggering device 6 is placed inside one of the supporting poles 13B. This mechanical triggering device 6 consists of a pivot supporting frame 60 and a trigger plank 61. The pivot supporting frame 60 is placed inside one of the supporting poles 13B At the central part near the top edge of each of the two sides of the top portion of the pivot supporting frame 60 that are facing each other, there is a pivot hole 62 The two pivot holes 62 respectively on the said two opposite sides are in line The two trigger plank pivot shafts 63 that protrude from the central part of the two narrower sides of the trigger plank 61 is respectively engaged into the two pivot holes 62 Thus, the trigger plank 61 can turn about the axis of the pivot

A device body 5 is installed on top of base body 10 This device body 5 includes a base ring 54 that is at the bottom of device body 5 and a center shell 50 that is on top of base ring 54 Base ring 54 is made of a material that is preferably translucent to visible light but opaque to UV light. The footprint of center shell 50 and base ring 54 matches the shape of the round platform 14 that is on top of base body 10. Thus after they are attached together and aligned, base 1 and device body 5 can be connected together by inserting screw bolts through the base ring connection hole 56 inside the base ring fixing block 55 on the rim of the base ring 54, and through the base connection hole 12 on the platform 14.

Furthermore, the preferred embodiment includes several cover lockers 52 intruding inside, as well as several slightly bulging reflection cup supporters 51 on the inner surface of the centre body 50 near the top. In the middle of each cover locker 52 there is a cover locking slot 53. Reflection cup supporter 51 is used to hold reflection cup 3 to mount it inside device body 5. Cover locking slot 53 is an irregular slat. One end of the slot is wider than the other. Thus the locking hook 24 beneath the top cover 2 can be placed inside it to lock the top cover 2 on top of the device body 5. The reflection cup 3 is installed inside device body 5. The reflection cup unit 30 has two connected portions: reflection cup ring 31 and reflection cup body 32. Reflection cup ring 31 is a cylinder with two indentations on its surface at the opposite ends of a diameter of the reflection cup 31. The two indentations form two reflection cup notches 33, which engage with the two intruding cover lockers 52 on the top portion of the center body 50. On the bottom of the reflection cup body 32 there is a cross-shaped, in this embodiment, hole which is the supporting pole passage hole 36 (as shown in Figure 3)

When the reflection cup 3 is installed into the device body 5, the supporting poles 13A and 13B pass through the supporting pole passage hole 36 to fit into the supporting pole holding cavities 34 inside the reflection cup body 32. The space between these supporting pole holding cavities 34 are disinfection cells 35 The objects to be disinfected will be placed here for disinfection.

In a preferred embodiment, an ultraviolet protective shield 4 comprises (a) a shield body 40, in this embodiment it is cylindrical, with four fixing arms 42A and 42B protruding horizontally from the top region of the outer surface of the cylindrical body to form a cross shape as view from above, (b) a stack of horizontal, ring-shaped, preferably thin, circular slats 41 strategically deployed at non-uniform vertical spacing below the lower circular rim of the shield body 40 and (c) a number of vertical links, preferably thin, protruding downwards from the bottom circular rim of the shield body 40 The various levels of horizontal circular slats 41 are linked together and kept at strategic vertical spacing by the said vertical links. The four fixing arms 42A and 42B of the protective shield 4 are mounted on top of the four supporting poles 13A and 13B, respectively, with the supporting pole extensions 17 priorly glued on top of the supporting poles 13A, as extended parts of supporting poles 13A

The ultraviolet lamp 47 has its two adjacent electrodes located at one end Each electrode has a connecting wire Lamp holder 48 embraces the top part of the ultraviolet lamp and its two electrodes. The lamp holder 48, together with the ultraviolet lamp 47, is inserted and fitted into the hollow center of the shield body 40 of the ultraviolet protective shield 4, with the whole length of the ultraviolet lamp 47 being surrounded by the stack of slats 41 of the ultraviolet protective shield 4. The two connecting wires of the ultraviolet lamp 47 are threaded through the central cavity of one of the supporting pole 13B to reach the circuit board 15 from where suitably conditioned electrical power for the ultraviolet lamp is obtained during disinfection periods. Since the electronics of the UV lamp driver is well-known in the trade, it is not described here. A protective shield cover 45 completely covers the top parts of ultraviolet protective shield 4 including its four fixing arms 42A and 42B. On two opposite arms of the protective shield cover 45 there are body connection holes 46, which align with the two body connection holes 43 on the fixing arms 42A, through which two fixing screws are applied

Top cover 2 is placed above the reflection cup 3 and on top of the center shell 50 There are several disinfection cell holes 21 on the cover body 20, which allow passage tubes 23 to extend into disinfection cells 35, leading objects to be disinfected into the disinfection cells 35. Close to the rim on the bottom side of the top cover 2 there are two downward extensions whose lower parts are the locking hooks 24, which lock respectively into the cover locking slots 53. The locking hooks 24 first enter the wider end of the cover locking slots 53. When top cover 2 is turned in the clockwise direction, the locking hooks 24 move towards the narrower ends of the cover locking slots 53 Thus the hooks on the bottom of locking hooks 24 press against the inner ceiling near the cover locking slots 53, locking the top cover 2 and the device body 5 together Additionally, on the bottom side of the top cover 2 near two of the disinfection cell holes 21, there are two downward extensions whose lower parts are the triggering hooks 22 After the top cover 2 and the center body 50 are locked together, the hooks at the bottom of the two triggering hooks 22 are respectively inserted into the triggering hook accepting slots at the top part of the two supporting poles 13B and the fixing arms 42B.

Figure 4 illustrates how the ultraviolet protective shield 4 of the ultraviolet disinfection device 100 prevents the ultraviolet light generated by the ultraviolet lamp 47 from leaking directly out of the device.. Figure 5 is a sectional elevation view of the ultraviolet protective shield 4. The ultraviolet protective shield 4 is made of a tough and strong material that is opaque to ultraviolet light and may be transparent, translucent or opaque to visible light. The stack of ring-shaped circular slats 41 of the ultraviolet protective shield 4 surrounds the ultraviolet lamp 47. The vertical thickness of the ring-shaped circular slats 41 is preferably as small as possible to minimize blockage of the ultraviolet light rays that shine into the disinfection cells 35, but thick enough to provide the required mechanical strength. To determine the appropriate thickness, factors such as the strength of the material, the fabrication method, the actual size of the shield and the overall cost allowed have to be considered. This embodiment employs injection molding of polycarbonate (PC) resin with stabilizer against UV added For an outer diameter of 20 mm for the slats 41, it is found that the optimal vertical thickness of the slats 41 is slightly less than 1 mm around the inner circumference of the circular slats and progressively reduced to about 0 5 mm around the outer circumference.

The various levels of horizontal slats 41 are linked together and kept at strategic vertical spacing by the said thin vertical links. The horizontal slats 41 are said to be strategically spaced because the spacing density is not uniform for all the various levels; densest at the top level and progressively less dense for the lower levels (see Figure 3) The specific horizontal width of each slat 41 in the stack and the specific vertical spacing of that slat from its neighbors are carefully selected so that all the slats in the stack together form a complete blockage against the ultraviolet light rays generated by the ultraviolet lamp 47 in all possible direct paths leading to the user's eyes through the disinfection cell holes 21 of the top cover 2, while the number of slats 41 in the stack is minimized so that the blockage effect is very little for the light rays that enter the disinfection cells 35 The vertical spacing of the different levels of the slats is not uniform because for any fixed point of view above any of the disinfection cell holes 21, the angle of view for a point at the ultraviolet lamp 47 varies with the vertical position of that point. By simple trigonometry, the optimal spacing of the different levels of the slats so as to form a good-enough complete shield against the direct leakage of ultraviolet light rays can be worked out As shown in Figure 4, the slats prevent the ultraviolet light 7 generated by the ultraviolet lamp 47 from leaking through the disinfection cell holes 21 into the user's eyes At the same time, most of the ultraviolet light is allowed to pass through the space between the slats 41 to reach the disinfection cells 35 and shine onto the major parts of the objects to be disinfected if the latter are inserted into the disinfection cells 35.

Figure 6 is a perspective sectional view along the axis 6-6 of Figure 1 In Figure 6, a supporting pole 13B is sectioned to show its interior where the mechanical triggering device 6 is accommodated. As already described in a previous paragraph, the mechanical triggering device 6 consists of the pivot supporting frame 60 and the trigger plank 61, The pivot supporting frame 60 keeps the trigger plank 61 in the proper position and provides a pivot axis for the trigger plank 61 to turn about After the top cover 2 is placed correctly to cover the device body 5, turning the top cover 2 in the clockwise direction will lock the top cover 2 and the device body 5 together. The two triggering hooks 22 will also be inserted respectively into the triggering hook accepting slots at the top part of supporting poles 13B and fixing arms 42B. As shown in Figure 7, triggering hook 22 pushes the top part 64 of the trigger plank 61 to the left. Thus the bottom part 65 of trigger plank 61 moves to the right because of the leverage action When it presses the micro-switch 18 inside the base 1, the circuit in the circuit board 15 is turned on, and the automatic disinfection program starts to cycle.

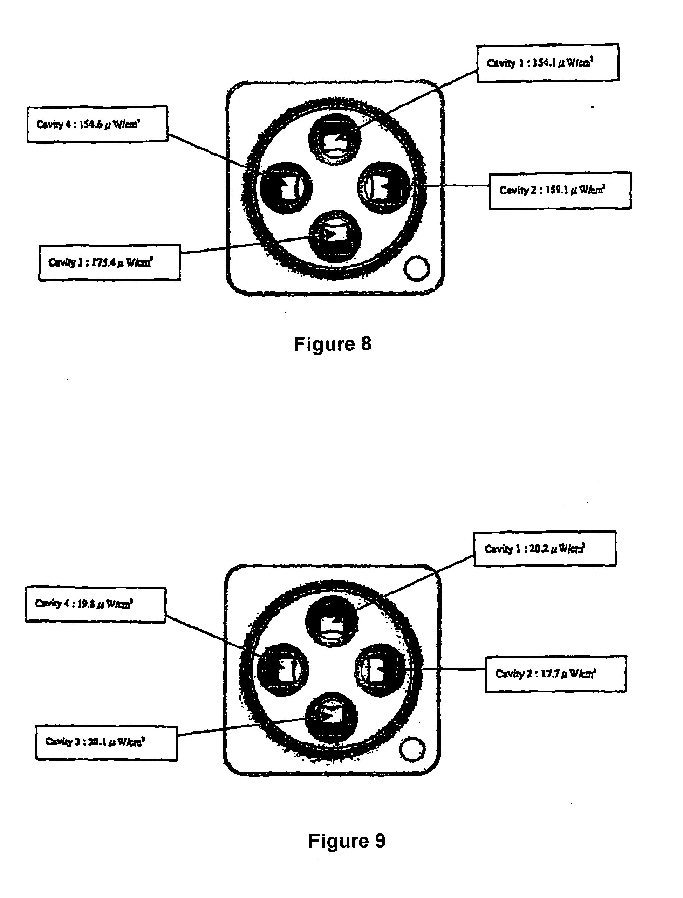

Figure 8 and Figure 9 illustrate, respectively, the strength of the ultraviolet light that leaks from this ultraviolet disinfection device 100 right outside the disinfection cell holes 21 before and after installation of the ultraviolet protective shield 4. In Figure 8, where the ultraviolet disinfection device 100 is without the ultraviolet protective shield 4, the ultraviolet strength readings right outside the disinfection cell holes 21 average to 160 µWcm⁻² But when the ultraviolet disinfection device 100 is with the ultraviolet protective shield 4 installed inside, the ultraviolet strength readings right outside the disinfection cell holes 21, as shown in Figure 9, average to 20 µWcm⁻². This demonstrates that the ultraviolet protective shield 4 can substantially reduce the leakage of the ultraviolet light from the ultraviolet disinfection device 100

Based on the abovementioned structure, ultraviolet disinfection device 100 utilizes a simple design to automatically disinfect long objects such as toothbrushes and chopsticks by simply inserting the objects into the disinfection cells 35 of this device 100 It is very easy to disinfect personal belongings. By utilizing the ultraviolet protective shield 4 around the ultraviolet lamp 47 with a shield body 40, this design both provides sufficient disinfection power and reduces substantially the leakage of ultraviolet light that may otherwise hurt the user's eyes. This ultraviolet disinfection device 100 is both convenient and safe to use, and is also good for the user's health

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A disinfection device (100) comprising:
a body portion (5) having an aperture configured to accept at least one object to be disinfected; and
at leat one germicidal lamp (47),
the device being provided with at least one protective shield (4) within the body portion, the shield having at least one slat (41), the device being configured such that the slat (41) is provided at a location between the lamp (47) and the aperture (21) along a line of sight direction between the lamp (47) and the aperture (21), thereby to reduce an amount of light from the lamp (47) that can pass through the aperture (21),
**characterised in that** the device (100) further comprises a top cover (2) that is supported by the body, the top cover (2) having at least one opening (21) for accepting the objects to be disinfected into at least one disinfecting cell; the top cover (2) having at least one triggering hook (22) for extending into the body (5) of the ultraviolet disinfection device (100); the at least one protective shield (4) surrounding the at least one lamp (47), the protective shield (4) being made of a material that is opaque to ultraviolet right the protective shield having a hollow body comprising a plurality of spaced parallel slats (41), the parallel slats (41) of a stack being connected by at least one link extending from the body of the protective shield (4), the center of each slat (41) having a hole larger than the diameter of the lamp (47), the hallow center of the body (5) of the protective shield (4) and the holes at the center of the layers of slats together forming a cavity for accommodating the lamp (47), at least a portion of the light-emitting length of the at least one germicidal lamp (47) being surrounded by the stack of slats (41),
wherein the protective shield (4) prevents direct leakage of light generated from the lamp (47) through the at least one opening (21) on the top cover (2).

2. A device as claimed in claim 1 wherein the shield (4) is configured to provide a sheath around the lamp (47).

3. A device as claimed in claim 1 or claim 2 wherein the slats (41) are provided with a major face of each slat substantial perpendicular to an axis of the shield (4).

4. A device as claimed in any preceding claim wherein the slats (41) are substantially coaxial.

5. A device as claimed in any preceding claim further comprising a reflection cup (3) mounted within the body of the device (100) beneath the top cover (2), the reflection cup (3) being removable by a user.

6. A device as claimed in claim 5 wherein the reflection cup (3) is arranged to surround at least a portion of objects to be disinfected and reflects at least a portion of light emitted from the at least one germicidal UV lamp (47) onto the objects to be disinfected.

7. A device as claimed in any preceding claim, further comprising an electronic circuit that controls operation of the lamp (47) such that, if activated, the lamp (47) is turned on for a predetermined duration at predetermined intervals.

8. A device as claimed in any preceding claim, wherein the density of vertical spacing of the horizontal slats (41) of the protective shield (4) is lower at an end of the shield distal the body (40) of the shield (4), and higher at an end of the shield proximate the body (40) of the shield.

9. A device as claimed in any preceding claim wherein:
the top cover (2) has at least one positioning and locking hook extending downwards from the bottom surface of the top cover (2);
there are provided cover locking slots (53) correspondingly located on the top surface of the body (50) to accept the at least one positioning and locking hook;
a shape of the cover locking slots (53) is irregular, being wider at one end and narrower at the other end;
when the top cover (2) is engaged with the body (50) of the device, the at least one positioning and locking hook is first inserted into the wider end of the slot (53);
when the top cover (2) is subsequently turned in the correct direction, the at least one positioning and locking hook moves towards the narrower end of the slot (53), resulting in the hook pressing on the ceiling near the narrower end of the slot (53), making the top cover (2) and the body (50) of the device locked together.

10. A device as claimed in and preceding claim wherein:
the body (50) of the device has at least one supporting pole (13A) extended upwards from the bottom;
the protective shield (4) has a least one fixing arm (42A) protruding outwards from the body of the protective shield;
the at least one fixing arm (42A) of the protective shield (4) is mounted on top of the at least one supporting pole (13A), and
the top cover (2) has at least one positioning and locking hook extending downwards from the bottom surface of the top cover (2).

11. A device as claimed in claim 5 or any of claims 6 to 10 as dependent on claim 5
wherein:
the body (5) of the device has a least one supporting pole (13A) extended upwards from the bottom;
the protective shield has a least one fixing arm (42A) protruding outwards from the body of the protective shield (4);
the at least one fixing arm (42A) of the protective shield (4) is mounted on top of the at least one supporting pole (13A);
the top cover (2) has at least one positioning and locking hook, extending downwards from the bottom surface of the top cover (2); and
the reflection cup (3) has at least one hole, appropriately shaped, at the bottom to allow passage of the at least one supporting pole (13A) through the at least one hole when the reflection cup (3) is placed in the body of the device (100).

12. A device as claimed in any preceding claim wherein the top cover (2) is removable.

13. A device as claimed in any preceding claim wherein a micro-switch (18) in the body of the device (100) is coupled to at least one of the at least one triggering hook of the removable top cover (2) such that when the removable top cover is removed or not placed properly, no power is supplied to the germicidal UV lamp (47).

14. A device as claimed in claim 13, further comprising:
a trigger plank (61) mounted within the body of the device by means of a pivot the axis of which passes through the trigger plank (61) somewhere around its midlength, the lower tip of the trigger plank (61) being coupled to the micro-switch (18) while the upper tip of the trigger plank (61) being coupled to the at least one triggering hook (22) of the removable top cover (2), placing the top cover properly on top of the body of the device (100) and then turning the top cover (2) in the correct direction resulting in making the at least one triggering hook (22) of the removable top cover (2) press against the upper tip of the trigger plank (61), consequently, because of leverage action, making the lower tip of the trigger plank (61) press against the micro-switch which then makes electrical connection to allow turning on of the at least one germicidal UV lamp (47), whereby when the top cover (2) is removed, improperly placed or not turned in the correct direction sufficiently to activate the micro-switch switch (18), no power is supplied to the germicidal UV lamp (47).

15. A device as claimed in claim 2 or any one of claims 3 to 14 depending through claim 2 wherein the sheath provided by the protective shield (4) protects the at least one germicidal UV lamp (47) from damage due to external mechanical forces exerted by the objects that are inserted into the body of the device (100).

16. A device as claimed in any preceding claim wherein the protective shield (4) reduces the ultraviolet light emitted from the device (100) eight fold.

17. A device as claimed in any preceding claim wherein the protective shield (4) reduces the ultraviolet light emitted from the device to 20 µWcm⁻².

## Patentansprüche

1. Desinfektionsvorrichtung (100), umfassend:
einen Gehäuseabschnitt (5) mit einer Öffnung, welche dazu ausgelegt ist, mindestens einen zu desinfizierenden Gegenstand aufzunehmen; und
mindestens eine Entkeimungslampe (47),
wobei die Vorrichtung mit mindestens einem Schutzschirm (4) innerhalb des Gehäuseabschnitts versehen ist, wobei der Schirm mindestens eine Lamelle (41) aufweist, wobei die Vorrichtung so ausgelegt ist, dass die Lamelle (41) an einer Stelle zwischen der Lampe (47) und der Öffnung (21) entlang einer Sichtlinienrichtung zwischen der Lampe (47) und der Öffnung (21) vorgesehen ist, um so eine Lichtmenge von der Lampe (47) zu reduzieren, die durch die Öffnung (21) hindurch treten kann,
**dadurch gekennzeichnet, dass** die Vorrichtung (100) ferner eine obere Abdeckung (2) umfasst, welche von dem Gehäuse getragen wird, wobei die obere Abdeckung (2) mindestens eine Öffnung (21) zur Aufnahme der zu desinfizierenden Gegenstände in mindestens eine Desinfektionszelle aufweist; wobei die obere Abdeckung (2) mindestens einen Auslösehaken (22) zum Hineinragen in das Gehäuse (5) der Ultraviolett-Desinfektionsvorrichtung (100) aufweist; wobei der mindestens eine Schutzschirm (4) die mindestens eine Lampe (47) umgibt, wobei der Schutzschirm (4) aus einem Material hergestellt ist, das für ultraviolettes Licht undurchlässig ist, wobei der Schutzschirm ein hohles Gehäuse aufweist, welches eine Mehrzahl voneinander beabstandeter paralleler Lamellen (41) umfasst, wobei die parallelen Lamellen (41) einer Säule durch mindestens ein sich von dem Gehäuse des Schutzschirms (4) erstreckendes Verbindungsstück verbunden sind, wobei die Mitte jeder Lamelle (41) ein Loch aufweist, welches größer ist als der Durchmesser der Lampe (47), wobei die hohle Mitte des Gehäuses (5) des Schutzschirms (4) und die Löcher in der Mitte der Lamellenschichten zusammen eine Ausnehmung zur Aufnahme der Lampe (47) bilden, wobei mindestens ein Abschnitt der Licht abstrahlenden Länge der mindestens einen Entkeimungslampe (47) von der Säule aus Lamellen (41) umgeben ist, wobei der Schutzschirm (4) das direkte Entweichen erzeugten Lichts von der Lampe (47) durch die mindestens eine Öffnung (21) an der oberen Abdeckung (2) hindurch verhindert.

2. Vorrichtung nach Anspruch 1, wobei der Schirm (4) dazu ausgelegt ist, eine Hülle um die Lampe (47) herum zu schaffen.

3. Vorrichtung nach Anspruch 1 oder Anspruch 2, wobei die Lamellen (41) so gestaltet sind, dass eine große Fläche jeder Lamelle im Wesentlichen rechtwinklig zu einer Achse des Schirms (4) verläuft.

4. Vorrichtung nach einem beliebigen vorangehenden Anspruch, wobei die Lamellen (41) im Wesentlichen koaxial zueinander angeordnet sind.

5. Vorrichtung nach einem beliebigen vorangehenden Anspruch, welche ferner eine innerhalb des Gehäuses der Vorrichtung (100) unterhalb der oberen Abdeckung (2) angebrachte Reflexionsschale (3) umfasst, wobei die Reflexionsschale (3) von einem Benutzer entfernbar ist.

6. Vorrichtung nach Anspruch 5, wobei die Reflexionsschale (3) so angeordnet ist, dass sie mindestens einen Teil zu desinfizierender Gegenstände umgibt, und mindestens einen Teil von der mindestens einen UV-Entkeimungslampe (47) abgestrahlten Lichts auf die zu desinfizierenden Gegenstände reflektiert.

7. Vorrichtung nach einem beliebigen vorangehenden Anspruch, welche ferner eine elektronische Schaltung umfasst, welche den Betrieb der Lampe (47) derart steuert, dass die Lampe (47) im aktivierten Zustand für eine vorbestimmte Dauer in vorbestimmten Intervallen eingeschaltet ist.

8. Vorrichtung nach einem beliebigen vorangehenden Anspruch, wobei die Dichte der vertikalen Anordnung der horizontalen Lamellen (41) des Schutzschirms (4) an einem zum Gehäuse (40) des Schirms (4) distalen Ende des Schirms niedriger und an einem zum Gehäuse (40) des Schirms proximalen Ende des Schirms höher ist.

9. Vorrichtung nach einem beliebigen vorangehenden Anspruch, wobei:
die obere Abdeckung (2) mindestens einen Positionier- und Verschlusshaken aufweist, welcher sich von der unteren Oberfläche der oberen Abdeckung (2) abwärts erstreckt;
entsprechend an der oberen Oberfläche des Gehäuses (50) angeordnete Schlitze (53) zum Verschluss der Abdeckung vorgesehen sind, um den mindestens einen Positionier- und Verschlusshaken aufzunehmen;
eine Form der Schlitze (53) zum Verschluss der Abdeckung unregelmäßig ist, wobei sie an einem Ende weiter und an dem anderen Ende enger ist;
wenn die obere Abdeckung (2) in das Gehäuse (50) der Vorrichtung eingreift, der mindestens eine Positionier- und Verschlusshaken zuerst in das weitere Ende des Schlitzes (53) eingesetzt wird;
wenn die obere Abdeckung (2) anschließend in die richtige Richtung gedreht wird, der mindestens eine Positionier- und Verschlusshaken sich zum engeren Ende des Schlitzes (53) hin bewegt, was dazu führt, dass der Haken auf die Decke nahe dem engeren Ende des Schlitzes (53) drückt, wodurch die obere Abdeckung (2) und das Gehäuse (50) miteinander verriegelt werden.

10. Vorrichtung nach einem beliebigen vorangehenden Anspruch, wobei:
das Gehäuse (50) der Vorrichtung mindestens einen sich vom Boden aufwärts erstreckenden Stützstab (13A) aufweist;
der Schutzschirm (4) mindestens einen aus dem Gehäuse des Schutzschirms nach außen herausragenden Befestigungsarm (42A) aufweist;
der mindestens eine Befestigungsarm (42A) des Schutzschirms (4) oben auf dem mindestens einen Stützstab (13A) angebracht ist, und
die obere Abdeckung (2) mindestens einen sich von der unteren Oberfläche der oberen Abdeckung (2) abwärts erstreckenden Positionierund Verschlusshaken aufweist.

11. Vorrichtung nach Anspruch 5 oder einem beliebigen der Ansprüche 6 bis 10, wenn diese von Anspruch 5 abhängen, wobei:
das Gehäuse (50) der Vorrichtung mindestens einen sich vom Boden aufwärts erstreckenden Stützstab (13A) aufweist;
der Schutzschirm (4) mindestens einen aus dem Gehäuse des Schutzschirms (4) nach außen herausragenden Befestigungsarm (42A) aufweist;
der mindestens eine Befestigungsarm (42A) des Schutzschirms (4) oben auf dem mindestens einen Stützstab (13A) angebracht ist;
die obere Abdeckung (2) mindestens einen sich von der unteren Oberfläche der oberen Abdeckung (2) abwärts erstreckenden Positionierund Verschlusshaken aufweist; und
die Reflexionsschale (3) am Boden mindestens ein geeignet geformtes Loch aufweist, um den Durchtritt des mindestens einen Stützstabes (13A) durch das mindestens eine Loch zu ermöglichen, wenn die Reflexionsschale (3) in dem Gehäuse der Vorrichtung (100) platziert wird.

12. Vorrichtung nach einem beliebigen vorangehenden Anspruch, wobei die obere Abdeckung (2) entfernbar ist.

13. Vorrichtung nach einem beliebigen vorangehenden Anspruch, wobei ein Mikroschalter (18) in dem Gehäuse der Vorrichtung (100) mit mindestens einem des mindestens einen Auslösehakens der entfernbaren oberen Abdeckung (2) derart gekoppelt ist, dass wenn die entfernbare obere Abdeckung entfernt oder nicht richtig platziert wird, der UV-Entkeimungslampe (47) kein Strom zugeführt wird.

14. Vorrichtung nach Anspruch 13, welche ferner Folgendes umfasst:
eine Auslöseplanke (61), welche innerhalb des Gehäuses der Vorrichtung mittels eines Zapfens angebracht ist, dessen Achse durch die Auslöseplanke (61) ungefähr auf der Hälfte ihrer Länge hindurch tritt, wobei die untere Spitze der Auslöseplanke (61) mit dem Mikroschalter (18) gekoppelt ist, während die obere Spitze der Auslöseplanke (61) mit dem mindestens einen Auslösehaken (22) der entfernbaren oberen Abdeckung (2) gekoppelt ist, wodurch die obere Abdeckung richtig oben auf das Gehäuse der Vorrichtung (100) platziert und dann die obere Abdeckung (2) in der richtigen Richtung gedreht wird, was dazu führt, dass der mindestens eine Auslösehaken (22) der entfernbaren oberen Abdeckung (2) gegen die obere Spitze der Auslöseplanke (61) drückt und demzufolge, aufgrund von Hebelwirkung, die untere Spitze der Auslöseplanke (61) gegen den Mikroschalter drückt, welcher dann die elektrische Verbindung herstellt, um das Einschalten der mindestens einen UV-Entkeimungslampe (47) zu ermöglichen, wodurch wenn die obere Abdeckung (2) entfernt, nicht richtig platziert oder nicht ausreichend in der richtigen Richtung gedreht wird, um den Mikroschalter (18) zu aktivieren, der UV-Entkeimungslampe (47) kein Strom zugeführt wird.

15. Vorrichtung nach Anspruch 2 oder einem beliebigen der Ansprüche 3 bis 14, wenn sie von Anspruch 2 abhängen, wobei die von dem Schutzschirm (4) geschaffene Hülle die mindestens eine UV-Entkeimungslampe (47) gegen Beschädigungen aufgrund äußerer mechanischer Kräfte schützt, welche von den Gegenständen ausgeübt werden, die in das Gehäuse der Vorrichtung (100) eingesetzt werden.

16. Vorrichtung nach einem beliebigen vorangehenden Anspruch, wobei der Schutzschirm (4) das von der Vorrichtung (100) abgestrahlte ultraviolette Licht achtfach reduziert.

17. Vorrichtung nach einem beliebigen vorangehenden Anspruch, wobei der Schutzschirm (4) das von der Vorrichtung abgestrahlte ultraviolette Licht auf 20 µWcm⁻² reduziert.

## Revendications

1. Un dispositif de désinfection (100) comportant :
une partie de corps (5) ayant une ouverture configurée pour accepter au moins un objet à désinfecter ; et
au moins une lampe germicide (47),
le dispositif étant équipé d'au moins un écran protecteur (4) dans la partie de corps, l'écran ayant au moins une lamelle (41), le dispositif étant configuré de façon que la lamelle (41) soit agencée à un endroit situé entre la lampe (47) et l'ouverture (21) le long d'une ligne de vision directe entre la lampe (47) et l'ouverture (21), afin de réduire une quantité de lumière émise par la lampe (47) qui peut passer à travers l'ouverture (21),
**caractérisé en ce que** le dispositif (100) comporte en outre un couvercle de sommet (2) qui est supporté par le corps, le couvercle de sommet (2) ayant au moins une ouverture (21) pour accepter les objets à désinfecter dans au moins une cellule de désinfection ; le couvercle de sommet (2) ayant au moins un crochet de déclenchement (22) pour s'avancer dans le corps (5) du dispositif de désinfection à ultra-violet (100) ; le au moins un écran protecteur (4) entourant la au moins une lampe (47), l'écran protecteur (4) étant réalisé en un matériau qui est opaque à la lumière ultra-violette, l'écran protecteur ayant un corps creux comprenant une pluralité de lamelles parallèles espacées (41), les lamelles parallèles (41) d'une pile étant reliées par au moins un lien s'étendant à partir du corps de l'écran protecteur (4), le centre de chaque lamelle (41) ayant un orifice plus grand que le diamètre de la lampe (47), le centre creux du corps (5) de l'écran protecteur (4) et les orifices situés au centre des couches de lamelles formant ensemble une cavité pour recevoir la lampe (47), au moins une partie de la longueur d'émission de lumière de la au moins une lampe germicide (47) étant entourée par la pile de lamelles (41), et où l'écran protecteur (4) empêche la fuite directe de la lumière émise par la lampe (47) par la au moins une ouverture (21) sur le couvercle de sommet (2).

2. Un dispositif selon la revendication 1, où l'écran (4) est configuré pour former une gaine autour de la lampe (47).

3. Un dispositif selon l'une des revendications 1 et 2, où les lamelles (41) sont agencées de façon qu'une face principale de chaque lamelle soit sensiblement perpendiculaire à un axe de l'écran (4).

4. Un dispositif selon l'une quelconque des revendications précédentes, où les lamelles (41) sont sensiblement coaxiales.

5. Un dispositif selon l'une quelconque des revendications précédentes comportant en outre une coupelle réflectrice (3) montée dans le corps du dispositif (100) en dessous du couvercle de sommet (2), la coupelle réflectrice (3) pouvant être démontée par un utilisateur.

6. Un dispositif selon la revendication 5, où la coupelle réflectrice (3) est agencée pour entourer au moins une partie des objets à désinfecter et réfléchit au moins une partie de la lumière émise par la au moins une lampe UV germicide (47) sur les objets à désinfecter.

7. Un dispositif selon l'une quelconque des revendications précédentes, comportant en outre un circuit électronique qui commande le fonctionnement de la lampe (47) de façon que, s'il est activé, la lampe (47) soit allumée pendant une durée prédéterminée à des intervalles prédéterminés.

8. Un dispositif selon l'une quelconque des revendications précédentes, où la densité d'espacement vertical des lamelles horizontales (41) de l'écran protecteur (4) est plus faible à une extrémité distale du corps (40) de l'écran (4), et plus élevée à une extrémité proximale du corps (40) de l'écran.

9. Un dispositif selon l'une quelconque des revendications précédentes, où :
le couvercle de sommet (2) a au moins un crochet de positionnement et de verrouillage s'étendant vers le bas à partir de la surface de fond du couvercle de sommet (2) ;
sont prévues des fentes de blocage du couvercle (53) situées en correspondance sur la surface de sommet du corps (50) pour recevoir le au moins un crochet de positionnement et de verrouillage ;
une forme des fentes de blocage du couvercle (53) est irrégulière, en étant plus large à une extrémité et plus étroite à l'autre extrémité ;
quand le couvercle de sommet (2) coopère avec le corps (50) du dispositif, le au moins un crochet de positionnement et de verrouillage est d'abord inséré dans l'extrémité la plus large de la fente (53) ;
quand le couvercle de sommet (2) est ensuite tourné dans le sens correct, le au moins un crochet de positionnement et de verrouillage se déplace vers l'extrémité plus étroite de la fente (53), permettant ainsi au crochet d'appuyer sur le plafond proche de l'extrémité la plus étroite de la fente (53), ce qui a pour effet de verrouiller ensemble le couvercle de sommet (2) et le corps (50) du dispositif.

10. Un dispositif selon l'une quelconque des revendications précédentes, où :
le corps (50) du dispositif a au moins une tige support (13A) s'élevant à partir du fond ;
l'écran protecteur (4) a au moins un bras de fixation (42A) émergeant vers l'extérieur à partir du corps de l'écran protecteur ;
le au moins un bras de fixation (42A) de l'écran protecteur (4) est monté au sommet de la au moins une tige support (13A), et
le couvercle de sommet (2) a au moins un crochet de positionnement et de verrouillage s'étendant vers le bas à partir de la surface de fond du couvercle de sommet (2).

11. Un dispositif selon la revendication 5 ou selon l'une quelconque des revendications 6 à 10 quand elle dépend de la revendication 5, où :
le corps (50) du dispositif a un moins une tige support (13A) s'élevant à partir du fond ;
l'écran protecteur a au moins un bras de fixation (42A) émergeant vers l'extérieur à partir du corps de l'écran protecteur (4) ;
le au moins un bras de fixation (42A) de l'écran protecteur (4) est monté au sommet de la au moins une tige support (13A) ;
le couvercle de sommet (2) a au moins un crochet de positionnement et de verrouillage s'étendant vers le bas à partir de la surface de fond du couvercle de sommet (2) ; et
la coupelle réflectrice (3) a, au fond, au moins un orifice de forme appropriée, pour permettre le passage de la au moins une tige support (13A) à travers le au moins un orifice quand la coupelle réflectrice (3) est placée dans le corps du dispositif (100).

12. Un dispositif selon l'une quelconque des revendications précédentes, où le couvercle de sommet (2) est amovible.

13. Un dispositif selon l'une quelconque des revendications précédentes, où un micro-interrupteur (18) dans le corps du dispositif (100) est couplé à au moins un du au moins un crochet de déclenchement du couvercle de sommet amovible (2) de façon que, quand le couvercle de sommet amovible est enlevé ou placé incorrectement, la lampe UV germicide (47) n'est pas alimentée en énergie.

14. Un dispositif selon la revendication 13, comportant en outre :
une came de déclenchement (61) montée dans le corps du dispositif au moyen d'un pivot dont l'axe passe à travers la came de déclenchement (61) sensiblement en son milieu, l'extrémité inférieure de la came de déclenchement (61) étant couplée au micro-interrupteur (18) tandis que l'extrémité supérieure de la came de déclenchement (61) est couplée à le au moins un crochet de déclenchement (22) du couvercle de sommet amovible (2), plaçant le couvercle de sommet correctement au sommet du corps du dispositif (100) et orientant alors le couvercle de sommet (2) dans le sens correct permettant à le au moins un crochet de déclenchement (22) du couvercle de sommet amovible (2) d'appuyer contre l'extrémité supérieure de la came de déclenchement (61), et en conséquence, en raison de l'effet de levier, permettant à l'extrémité inférieure de la came de déclenchement (61) d'appuyer contre le micro-interrupteur qui établit alors la connexion électrique pour permettre l'allumage de la au moins une lampe UV germicide (47), de façon que, quand le couvercle de sommet (2) est enlevé, incorrectement placé ou non suffisamment tourné dans le sens correct pour actionner le commutateur du micro-interrupteur (18), la lampe UV germicide (47) n'est pas alimentée en énergie.

15. Un dispositif selon la revendication 2 ou l'une quelconque des revendications 3 à 14 quand elle dépend de la revendication 2, où la gaine réalisée par l'écran protecteur (4) protège la au moins une lampe UV germicide (47) contre des dommages dus à des forces mécaniques extérieures exercées par les objets qui sont insérés dans le corps du dispositif (100).

16. Un dispositif selon l'une des revendications précédentes, où l'écran protecteur (4) réduit de huit fois la lumière ultraviolette émise à partir du dispositif (100).

17. Un dispositif selon l'une des revendications précédentes, où l'écran protecteur (4) réduit à 20 µWcm⁻² la lumière ultraviolette émise à partir du dispositif.
